# EUROPEAN PATENT SPECIFICATION

(11) **EP 2 571 489 B1**
(45) Date of publication and mention of the grant of the patent: **06.08.2014**
(21) Application number: 11786988.3
(22) Date of filing: 12.05.2011
(51) Int. Cl.: A61K 9/107, A61K 31/216, A61K 47/14, A61P 23/00

(54) **INJECTABLE EMULSION OF SEDATIVE HYPNOTIC AGENT**
INJIZIERBARE EMULSION AUS EINEM SEDATIVEN HYPNOSEMITTEL
EMULSION INJECTABLE D'UN AGENT HYPNOTIQUE SÉDATIF

(30) Priority: 13.05.2010 US 334208 P
(43) Date of publication of application: 27.03.2013
(73) Proprietor: AstraZeneca AB, 151 85 Södertälje (SE)
(72) Inventor: BOOTH, Jonathan, Macclesfield Cheshire SK10 2NA (GB); DIXON, Leigh, Macclesfield Cheshire SK10 2NA (GB); WASHINGTON, Clive, Macclesfield Cheshire SK10 2NA (GB)
(86) International application number: PCT/SE2011/050602
(87) International publication number: WO 2011/149412

(56) References cited:
- EP-A1- 1 875 901
- EP-A1- 1 875 901
- EP-A2- 0 211 258
- WO-A2-2005/009420
- WO-A2-2005/009420
- US-A- 4 711 902
- US-A1- 2003 153 554
- US-A1- 2003 153 554

## Description

The present invention relates to novel pharmaceutical formulations comprising a substituted phenylacetic acid ester compound, which is useful as a short-acting sedative hypnotic agent for anesthesia and sedation, in an oil-in-water emulsion suitable for administration by injection, and to processes for the preparation of the formulations and the uses of the formulations in medical treatment of a mammal.

### Background of the Invention

Sedative hypnotic agents are widely used for the induction and maintenance of general anesthesia, for sedation during surgical or diagnostic procedures, and for sedation of patients in intensive care. U.S. Patent No. 6,887,866 discloses the compound [3-ethoxy-4-[(*N,N*-diethylcarbamido)methoxy]phenyl]acetic acid *n*-propyl ester with structural formula 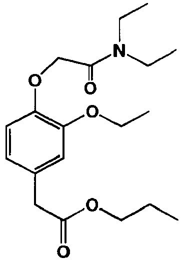 (hereinafter referred to as Compound A). Compound A is a useful short-acting sedative hypnotic agent. Among other properties, Compound A is expected to be pharmacokinetically responsive, providing shorter and more predictable duration of action than other sedative hypnotic agents.

Agents for sedation and anaesthesia are frequently administered by intravenous injection, a form of administration for which the agents need to be in a water-miscible form. Compound A, however, is an oleaginous compound with a water solubility of about 2 mg/ml. Such a compound is considered "slightly soluble" in the solubility definitions in General Notices 5.30 of the United States Pharmacopeia, USP33-NF28, published by the United States Pharmacopeial Convention Inc., Rockville, MD. In the case of Compound A, this solubility is not optimal for a therapeutically useful dose to be prepared simply by dissolution in water. The preparation of slightly soluble medicinal compounds for intravenous administration has been the subject of considerable investigation, but remains a significant challenge in the development of new therapeutic agents. Several drug delivery systems have been investigated for such compounds. In the present application, emulsions, in which droplets of oil, in which the drug is itself dissolved, are dispersed in an aqueous medium with the aid of an emulsifier and other suitable excipients and processing. A similar method is used for the commercial formulation of propofol, (2,6-diisopropyl-phenol), which is commercially available as Diprivan^{®} Injectable Emulsion at concentrations of 1% and 2%. Oil-in-water emulsions comprising propofol are described in WO 96/29064.

Intravenous emulsions should have a very small droplet size to circulate in the bloodstream without causing capillary blockage and embolisation. These size limits are typified by USP33-NF28 General Chapter <729> for Globule Size Distribution in Lipid Injectable Emulsions, hereinafter referred to as USP <729>, which defines universal limits for (1) mean droplet size not exceeding 500 nm or 0.5 µm and (2) the population of large-diameter fat globules, expressed as the volume-weighted percentage of fat greater than 5 µm (PFAT5) not exceeding 0.05%, irrespective of the final lipid concentration.

Emulsion formulations must be physically stable. The droplet size limits defined in USP <729> apply throughout the assigned shelf life, which for a commercial pharmaceutical formulation would typically extend to 2-3 years or longer. All true emulsions are thermodynamically unstable and may over time undergo a range of processes which tend to increase the droplet size. These include direct droplet coalescence, when two droplets collide and form a single new droplet, and aggregation, in which droplets adhere together to form larger masses. Aggregation may in some cases be a precursor of further coalescence into larger droplets. Ultimately these processes may result in free oil being visible on the emulsion surface, or large aggregates rising to the surface of the container, a phenomenon known as 'creaming'. Droplet size measurements such as those defined in USP<729> can measure the initial increases in size, and hence are predictive of emulsion physical stability, at early times, long before the formulation shows macroscopic visible changes.

Emulsion formulations must also be chemically stable. The drug substance may degrade; for example, lipophilic drugs will partition into the oil phase, which will confer some degree of protection, but hydrolytic degradation may still occur at the oil-water interface. Possible chemical degradation within parenteral fat emulsions includes oxidation of unsaturated fatty acid residues present in triglyceride and lecithin, and hydrolysis of phospholipids leading to the formation of free fatty acids (FFA) and lysophospholipids. Such degradants lower pH, which may then promote further degradation. Thus, pH should be controlled during manufacture and parenteral emulsion formulations may include a buffering agent to provide additional control. Any decrease in pH over the assigned shelf-life may be indicative of chemical degradation.

In the case of charge-stabilized emulsions, such as those in which lecithin is the emulsifier, the stabilizing charge may vary with pH. Consequently changes in pH due to chemical degradation may also accelerate physical degradation. If the emulsion is sterically stabilized, for example by a poly(oxyethylene) surfactants, changes in pH will generally have little effect on emulsion stability.

WO 2005/009420 discloses an injectable emulsion for Compound A comprising a water immiscible solvent, an emulsifier, a tonicity modifier, a pH buffering agent and water. The inclusion of histidine is claimed to have an improved effect on the stability of the emulsion, specifically with regard to pH, chemical formulation and particle size. The water immiscible solvent is a plant-based oil such as soybean oil or safflower oil, and the emulsifier used is lecithin derived from egg yolk. The emulsion has a mean droplet size of 330 nm. No means for sterilising the emulsion is mentioned, nor is the fraction of oil present as large droplets disclosed.

Emulsions for intravenous use must be sterile, and a process for sterilisation is an essential part of any intravenous formulation. In general, terminal sterilization by autoclave is a preferred route, and is, for example, the process by which Diprivan^{®} Injectable Emulsion is sterilized. However, in the case of Compound A, autoclave sterilization of the formulations disclosed in WO 2005/009420 resulted in extensive coalescence and formation of free oil.

An alternate method of sterilizing emulsions is to pass them through a filter that is sufficiently small to retain bacteria and spores, but that will allow emulsion droplets to pass. The nominal pore size of such a filter is 0.2 µm (200 nm). The emulsion disclosed in WO 2005/009420 could not be sterilised in this manner since its mean droplet diameter of 330 nm is too large to pass through such a filter. Filtration has never been used to sterilize a commercial intravenous emulsion product since the emulsion droplets generally cannot be made sufficiently small to pass through the filter. Indeed, the mean droplet size limit of 0.5 µm specified in USP <729> is too coarse to allow filtration to be used for sterilization.

Sterility may be assessed by means of a sterility test, such as that described in USP33-NF28 General Chapter <71> or the equivalent procedures described in the European and Japanese Pharmacopoeia.

It should be understood that this application relates only to true emulsions and not to microemulsions. These two systems are frequently confused in the literature due to careless use of terminology. A microemulsion is formed spontaneously, without homogenization, when appropriate oils, surfactants, and water are mixed, and has a small droplet size which frequently permits it to pass through sterilizing-grade filters. The emulsions presented in this application do not form spontaneously, and require the use of a high-shear homogenization step to achieve a droplet size which is sufficiently small for filter sterilization and intravenous administration.

In the present application, substantial research was conducted to identify a formulation and process that will allow Compound A to be incorporated into an emulsion with a sufficiently small droplet size to allow sterilization by filtration and, as a consequence, also satisfies the requirements of USP <729> throughout the designated shelf life of the formulation.

### Summary of the Invention

The present invention provides for an emulsion whereby physical stability can be improved markedly by use of an appropriate lecithin or by polymeric stabilisers.

The pharmaceutical formulation of the present invention may particularly comprise soybean-derived lecithin. It has been found that this lecithin improves the storage stability of the emulsion, more than e.g. egg-derived lecithin does. The soybean-derived lecithin when combined with medium chain triglyceride (MCT) oil of relatively low viscosity and processed using optimized conditions gives rise to a more stable emulsion with a small droplet size and improved resistance to droplet coalescence. This droplet size is small enough to allow sterilization by filtration instead of or prior to autoclaving.

### Brief Description of the Drawings

**Figure 1** shows a typical chromatogram for the determination Compound A assay and impurities content. The vertical axis represents response and the horizontal axis represents retention time (minutes).
**Figure 2** shows a typical 500MHz ¹H NMR spectrum for the determination of free fatty acid (FFA) content of Compound A emulsion. The vertical axis represents signal intensity and the horizontal axis represents chemical shift in parts per million (ppm).
**Figure 3** shows an expanded 500MHz ¹H NMR spectrum for the region of the FFA methylene proton signal in the determination of FFA content of Compound A emulsion.
The vertical axis represents signal intensity and the horizontal axis represents chemical shift in parts per million (ppm).
**Figure 4** shows a typical ³¹P NMR spectrum for the determination of lysophosphatidocholine content of Compound A emulsion. The vertical axis represents signal intensity and the horizontal axis represents chemical shift in parts per million (ppm).
**Figure 5** shows an expanded ³¹P NMR spectrum of Compound A emulsion. The vertical axis represents signal intensity and the horizontal axis represents chemical shift in parts per million (ppm).

### Description of Embodiments

When describing the formulations and methods of the invention, the following terms have the following meanings, unless otherwise indicated.

As used herein, the phrase "hypnotic agent" refers generally to a compound that promotes sleep. As used in pharmacology, the phrase "hypnotic agents" describe agents used to induce or maintain anaesthesia, sedation, or sleep.

As used herein, the term "anaesthesia" means a loss of consciousness, sensation, or awareness resulting from pharmacological depression of nerve function.

As used herein, the term "sedation" means the calming of mental excitement or abatement of physiological function by administration of a drug.

As used herein, the phrase "effective amount" means that amount which is sufficient to induce or maintain anaesthesia or sedation when administered to a mammal. The effective amount will vary depending on the subject and the manner of administration, and may be determined routinely by one of ordinary skill in the art.

As used herein, the term "analgesic" means a compound that relieves pain by altering perception of nociceptive stimuli without producing significant anaesthesia or loss of consciousness.

As used herein, the term "opioid" means a synthetic narcotic that has opiate-like activities (e.g., analgesia), but is not derived from opium.

As used herein, the term "paralytic agent" means a compound that causes paralysis of the affected skeletal muscles by blocking neuromuscular transmission at the neuromuscular junction.

As used herein, the phrase "short-acting" refers to agents that are pharmacokinetically responsive. When short-acting agents are administered by infusion, the effects of the agents cease promptly upon termination of the infusion.

As used herein, the term "isotonic" means having an osmotic pressure equal or similar to that of physiological fluids. Body fluids normally have an osmotic pressure that often is described as corresponding to that of a 0.9 % (w/v) aqueous solution of sodium chloride.

As used herein, the term "buffer" or "buffered" means a solution containing both a weak acid and its conjugate base, whose pH changes only slightly upon addition of acid or base. As used herein, the phrase "buffering agent" means a species whose inclusion in a solution provides a buffered solution.

As used herein, the term "about" means ± 5% of the value being modified.

The amounts as mentioned in this description are defined as ranges and are meant to include any numeric value within the interval, including the end-points. Thus, a range from 0 to about 5, means any number from 0 to about 5, such as 0, 1, 3, 4 and 5, but also, for example, 0.22, 1.28 and 4.67.

Weight % (wt%) is expressed as the percentages of the total weight of the pharmaceutical formulation.

The present invention provides for pharmaceutical formulations comprising the short-acting sedative agent [3-ethoxy-4-[(*N,N-*diethylcarbamido)methoxy]phenyl]acetic acid *n*-propyl ester (Compound A) as the active agent in a lipid emulsion formulation suitable for administration by injection. In some embodiments, the pharmaceutical formulation is an emulsion.

The amount of Compound A used in the pharmaceutical formulations of the present invention may vary from about 0.25 wt% to about 25 wt%. In one embodiment, the amount ranges from about 0.25 wt% to about 15 wt%. In another embodiment, the amount ranges from about 0.25 wt% to about 10 wt%. In a further embodiment, the amount ranges from about 0.5 wt% to about 10 wt%. In yet a further embodiment, the amount ranges from about 0.25 wt% to about 5 wt%. In yet a further embodiment, the amount ranges from about 5.5 wt% to about 10 wt%. In yet a further embodiment, the amount ranges from about 8 wt% to about 10 wt%.

In some embodiments, the pharmaceutical formulations comprise a water-immiscible solvent in which the active agent is miscible to a reasonable extent, such that a significant concentration of active agent can be achieved in the emulsion. The water-immiscible solvent may be a plant or animal derived oil including, but not limited to, soybean oil, sunflower oil, safflower oil, castor oil, sesame oil, corn oil, coconut oil, olive oil, and any mixture thereof. Alternatively, the solvent is a medium chain or long chain triglyceride or a mixture thereof, a hydrogenated plant-based oil, or material such as fish oil, vitamin E or squalane, or a single component fractionated from one of these natural oils. Semisynthetic oils such as acetylated monoglycerides (Myvacet) may also be used. In one embodiment of the invention, the water-immiscible solvent is medium chain triglycerides (MCT), pharmacopoeial grade.

The amount of water-immiscible solvent used in the emulsions of the present invention may vary from about 0.1 wt% to about 50 wt%. In another embodiment, the amount ranges from about 1 wt% to about 25 wt%. In a further embodiment, the amount ranges from about 5 wt% to about 15 wt%. In a further embodiment, the amount ranges from about 5 wt% to about 14 wt%. In a further embodiment, the amount ranges from about 5 wt% to about 13 wt%. In a further embodiment, the amount ranges from about 5 wt% to about 12 wt%. In a further embodiment, the amount ranges from about 5 wt% to about 11 wt%. In a further embodiment, the amount ranges from about 5 wt% to about 10 wt%. In a further embodiment, the amount ranges from about 5 wt% to about 9 wt%.

The pharmaceutical formulations also comprise an emulsifier. Useful emulsifiers include, but are not limited to, polyethoxylated ethers, esters and oils such as macrogols, and phospholipids of which lecithin is an example, castor oil and its derivatives (cremophor, macrogol 15 hydroxystearate), polyoxyethylene-polyoxypropylene copolymers and polyoxyethylene derivatives of vitamin E such as tocopheryl polyethylene glycol 1000 succinate. In one embodiment, the emulsifier is lecithin.

The term "lecithin" is used in its art-recognised manner (USP33-NF28). Lecithin includes a complex mixture of acetone-insoluble phosphatides, of which phosphatidylcholine is a significant component. The term lecithin is also used as a synonym for phosphatidylcholine. Useful lecithins include, but are not limited to, eggyolk-, soybean-, and corn-derived lecithin. In one embodiment, the emulsifier is lecithin, such as soybean-derived lecithin.

The amount of emulsifier used in the emulsions of the present invention may vary from about 0.001 wt% to about 15 wt%. In one embodiment, the amount ranges from about 0.01 wt% to about 10 wt%. In another embodiment, the amount ranges from about 0.01 wt% to about 5 wt%. In a further embodiment, the amount ranges from about 0.1 wt% to about 2.5 wt%.

The pharmaceutical formulations also comprise a tonicity modifier to make the formulation isotonic with blood. Suitable tonicity modifiers include, but are not limited to, glycerol, sorbitol, xylitol, mannitol, dextrose, glucose, polyethylene glycol, propylene glycol, sucrose, inorganic salts such as sodium chloride and lactose. In one embodiment, the tonicity modifier is glycerol. In another embodiment the emulsifier is a polymeric surfactant and an inorganic salt is the tonicity modifier.

The amount of tonicity modifier used in the emulsions of the present invention may vary from about 0.001 wt% to about 10 wt%. In one embodiment, the amount ranges from about 0.01 wt% to about 5 wt%. In another embodiment, the amount ranges from about 0.01 wt% to about 3 wt%. In a further embodiment, the amount ranges from about 0.1 wt% to about 2.5 wt%.

The pharmaceutical formulations also comprise water, in an appropriate amount.

The pharmaceutical formulations are formulated to be at physiologically compatible pH, which is typically defined as the range from about 5.0 to about 9.0. The pharmaceutical formulations may have a pH in the range of from about 6.5 to about 7.5. The pH is adjusted by the addition of a base, for example NaOH or NaHCO₃.

The pharmaceutical formulations optionally comprise a stabilizer that can alternately be considered a co-emulsifier. Stabilizers are beneficial in promoting the physical stability of the emulsion over time, i.e. in retarding separation of the oil and aqueous phases, on storage. Useful stabilizers include, but are not limited to, fatty acids such as oleic acid and its sodium salt, cholic acid and deoxycholic acids, cationic lipids such as stearylamine, and anionic stabilizers such as phosphatidylethanolamines, phosphatidylserines, phosphatidic acid and phosphatidylglycerol. In one embodiment, the stabiliser is oleic acid.

When present, the amount of stabilizer used in the emulsions of the present invention may vary from about 0.001 wt% to about 5 wt%. In one embodiment, the amount ranges from about 0.001 wt% to about 2 wt%. In another embodiment, the amount ranges from about 0.001 wt% to about 1 wt%. In a further embodiment, the amount ranges from about 0.001 wt% to about 0.5 wt%. In yet a further embodiment, the amount ranges from about 0.001 wt% to about 0.1 wt%. In yet another embodiment, the amount ranges from about 0.001 wt% to about 0.05 wt%.

The pharmaceutical formulations may optionally further comprise pH buffering agents such as, for example, sodium phosphate, sodium citrate, sodium bicarbonate, TRIS and amino acid buffers such as histidine.

When present, the amount of buffering agent is from about 0.01 wt% to about 10 wt%. In one embodiment, the amount ranges from about 0.01 wt% to about 5 wt%. In another embodiment, the amount ranges from about 0.01 wt% to about 2.5 wt%. In a further embodiment, the amount ranges from about 0.1 wt% to about 1 wt%.

The pharmaceutical formulations may also optionally comprise additives. Suitable additives include, but are not limited to preservatives such as phenol, derivatives of phenol such as methylparaben and propylparaben, benzoic acid and its salts, benzyl alcohol, cresol, chlorocresol, chlorobutanol, sodium metabisulphite, sodium sulphite, antimicrobial agents such as ethylenediaminetetraacetic acid and its salts, and antioxidants such as ascorbic acid and vitamin E.

When present, the amount of additives is from about 0.001 wt% to about 10 wt%. In one embodiment, the amount ranges from about 0.001 wt% to about 5 wt%. In a further embodiment, the amount ranges from about 0.001 wt% to about 1 wt%.

In one embodiment, the present invention provides a pharmaceutical formulation comprising an active agent, a water-immiscible solvent, a stabilizer, a tonicity modifying agent, an emulsifier and water, whereby the emulsifier is a soybean-derived lecithin, and wherein the formulation optionally further comprises a buffering agent and/or an additive.

In a further embodiment, the pharmaceutical formulation comprises Compound A, a water-immiscible solvent, a stabilizer, a tonicity modifying agent, an emulsifier, and water, whereby the emulsifier is soybean-derived lecithin, and wherein the formulation optionally further comprises a buffering agent and/or an additive.

In some embodiments, the formulation does not comprise histidine and/or a preservative / antimicrobial agent.

In another embodiment the pharmaceutical formulation comprises:

| *Component* | *weight%* |
|---|---|
| Compound A | 1 to 10 |
| Water-immiscible solvent | 5 to 15 |
| Stabilizer | 0 to 2 |
| Emulsifier | 1 to 5 |
| Tonicity modifier | 0 to 5 |
| Base | to pH 4.5 - 8.0 |
| Remaining water for injection | up to 100% |

and wherein the formulation optionally further comprises a buffering agent and/or an additive.

In another embodiment, the pharmaceutical formulation comprises:

| *Component* | *weight%* |
|---|---|
| Compound A | 1 to 10 |
| Medium chain Triglycerides | 5 to 15 |
| Oleic acid | 0 to 2 |
| Soybean-derived lecithin | 1 to 5 |
| Glycerol | 0 to 5 |
| Sodium hydroxide | to pH 7 |
| Remaining water for injection | up to 100% |

and wherein the formulation optionally further comprises a buffering agent and/or an additive.

In another embodiment, the pharmaceutical formulation comprises:

| *Component* | *weight%* |
|---|---|
| Compound A | 6 to 10 |
| Medium chain Triglycerides | 5 to 9 |
| Macrogol 15 Hydoxystearate | 0 to 4 |
| Poloxamer 188 | 0 to 4 |
| Citric acid buffer | pH 4.6 to 6 up to 100% |

In another embodiment, the pharmaceutical formulation comprises:

| *Component* | *weight%* |
|---|---|
| Compound A | 3 to 9 |
| Medium chain Triglycerides | 6 to 12 |
| Soybean-derived lecithin | 0.3 to 3 |
| L-Histidine | 0.1 to 1 |
| Disodium edetate | 0.001 to 0.1 |
| Glycerol | 1 to 2.5 |
| Remaining water for injection | up to 100% |

A further embodiment relates to the pharmaceutical formulations above which are sterilized by filtration.

### Medical use

The pharmaceutical formulations of the present invention can be used for the induction and/or maintenance of general anesthesia, for the initiation and/or maintenance of conscious sedation with patients spontaneously breathing, and for the induction and/or maintenance of sedation for intubated, mechanically ventilated patients. Thus, the invention also includes a method of inducing or maintaining anesthesia or sedation in a mammal, the method comprising administering to the mammal an effective amount of a pharmaceutical formulation of the invention.
Another embodiment relates to the use of the pharmaceutical formulations of the invention in the manufacture of a medicament for use in inducing or maintaining anesthesia or sedation in a mammal.

The amount of the active agent required for use in the methods of the invention will vary with the method of administration, the age and condition of the patient, and the degree of anesthesia or sedation required, and will be ultimately at the discretion of the attendant physician or clinician.

In general, the formulations can be administered as an initial bolus dose to produce anaesthesia or sedation, followed by a continuous infusion of formulation at a rate that is sufficient to achieve and maintain the level of anaesthesia or sedation desired. Alternatively, a continuous infusion of a formulation of the present invention can be used to maintain anaesthesia or sedation following induction or induction and maintenance with another sedative hypnotic agent, (e.g. propofol, a barbiturate, such as nembutal® (pentobarbital sodium) or brevital® sodium (methohexital sodium), or a benzodiazepine, such as valium®).
For example, a suitable bolus dose of the present agent for a human patient will typically be in the range of from about 0.1 milligrams/kilogram (mg/kg) to about 50 mg/kg, from about 0.5 mg/kg to about 20 mg/kg, or from about 0.8 mg/kg to about 1.2 mg/kg. The rate of infusion will typically be in the range from about 0.3 milligrams/kilogram/hour (mg/kg/hr) to about 300 mg/kg/hr, or from about 10 mg/kg/hr to about 60 mg/kg/hr. A bolus dose will be administered over a short period, for example one minute, whereas infusion may be continued for a prolonged period, for example 14 hours.

The formulations of the invention can also be administered in combination with other therapeutic agents, such as, for example, other sedative hypnotic agents, analgesics (e.g. an opioid such as the µ-opioid agonist remifentanil, fentanyl, sulfentanil, or alfentanil), or paralytic agents, such as atracurium besylate or pancuronium bromide. Accordingly, the formulations of the invention can optionally further comprise another therapeutic agent, for example, a sedative hypnotic agent, analgesic, or paralytic agent. Similarly, the therapeutic methods of the invention can also optionally comprise administering another therapeutic agent (e.g. a sedative hypnotic agent, analgesic, or paralytic agent) to the mammal.

The invention further provides a formulation according to the invention for use in therapy, the use of a formulation according to the invention for inducing or maintaining anaesthesia in a mammal, and the use, as above, wherein the use further comprises administering to the mammal a therapeutically effective amount of a therapeutic agent selected from a sedative hypnotic agent, an analgesic, and a paralytic agent.

Various active agents may be administered in the emulsion according to the present invention. Suitable agents are those capable of being administered parenterally in an oil-in-water emulsion. Typically such agents are lipophilic compounds and may for example be anti-fungal agents, anaesthetics, antibacterial agents, anti-cancer agents, anti-emetics, agents acting on the nervous system such as propofol, diazepam, steroids, barbiturates and vitamin preparations.

### Method of preparation

The active agent, [4-[*(N,N-*diethylcarbamoyl)methoxy]-3-ethoxyphenyl]acetic acid propyl ester, Compound A, can be synthesized as described in, for example, U.S. Patent No. 6,887,866.

The pharmaceutical formulations of the present invention may be prepared by combining the water phase components, i.e. the emulsifier, tonicity modifying agent, water and optionally an additive and/or a buffering agent (aqueous phase). The aqueous mixture is then dispersed using a homogenizer. The active agent is combined with the water-immiscible solvent and stabilizing agent, and stirred until homogenously dispersed to produce an oil phase mixture (oil phase). The aqueous phase is combined with the oil phase, under homogenization, to produce a coarse emulsion premix. This premix is introduced into a homogeniser, which may be a microfluidiser, previously primed with water, and homogenized at pressure. Output from the homogeniser is initially run to waste to remove priming water, and then collected in a clean vessel when the stream becomes completely cloudy. The homogeniser cycle is repeated to sufficiently reduce oil droplet size. The emulsion is than allowed to cool to ambient temperature whereafter the pH is adjusted to greater than about 7, if needed, with a base. The pharmaceutical formulation is then passed through a filter system at room temperature, and / or autoclaved, to achieve sterilization.

The pressure used for the homogenisation may vary. The pressures may be between 6000 and 24,000 psi.

The filters used to achieve sterilisation may be chosen by the skilled artisan and will have nominal pore size of 0.2 µm.

For large scale production the method above may need to be modified. Accordingly, the invention further provides for a method of preparing pharmaceutical formulations, the method comprising combining an emulsifier, optionally a stabilizing agent, a tonicity modifier, water, and optionally a buffering agent and/or an additive, to form an aqueous phase solution; adjusting the pH of the aqueous phase solution with base to a pH of greater than about 7; combining [4-[(N,N-diethylcarbamoyl)methoxy]-3-ethoxyphenyl]acetic acid propyl ester with a water-immiscible solvent to form a lipid phase mixture; adding the aqueous phase mixture to the lipid phase and emulsifying the resulting mixture to form the pharmaceutical formulation. In a specific embodiment of the method, the water-immiscible solvent is MCT, and the pH is adjusted after emulsification to a target of 7; and then sterilizing the pharmaceutical formulation by filtration and / or autoclaving.

A skilled practitioner could combine these materials in a different order and using different processing equipment to achieve the desired end result.

As described above and in the appended examples, one of the principal benefits of including soybean-derived lecithin in the emulsion of the invention is production of droplets that are small enough to permit sterilization by filtration. These benefits may also be realized using polymeric stabilized emulsions.

Accordingly, the invention further provides a method of preparing an emulsion having a pH of about 7, the emulsion comprising [4-[(*N,N*-diethylcarbamoyl)methoxy]-3-ethoxyphenyl]acetic acid propyl ester, a water-immiscible solvent, and water, the method comprising including a weight percentage of soybean-derived lecithin in the range from about 1 wt% to about 5 wt%, and which emulsion is sterilised by way of filtration.

In all embodiments, the production process, including sterilization by filtration, results in a product that is free from viable microorganisms and complies with appropriate pharmacopoeial tests for sterility.

In a further embodiment, the emulsion may also be sterilised by autoclave using a standard pharmacopoeial cycle, as an adjunct to, or an alternative to, filtration.

In order that the invention disclosed herein may be more efficiently understood, examples are provided below. It should be understood that these examples are for illustrative purposes only and are not to be construed as limiting the invention in any manner.

### Examples

The pharmaceutical formulation was produced at different scales using methods described in detail in the examples below. Analytical data were obtained using the same general methods as follows:

### Compound A assay and impurity content by Liquid Chromatography

Compound A, Compound A acid, and total impurities contents were determined using High Performance Liquid Chromatography (HPLC). The sample solution was prepared by dilution of Compound A emulsion with acetonitrile to a target concentration of 1.5mg/mL Compound A. 10µL sample was injected into a mobile phase comprising 0.1% trifluoroacetic acid in water (Eluent A) / 0.1% trifluoroacetic acid in acetonitrile (Eluent B), as defined by the gradient program in the table below.

| Gradient programme | Time (mins) | %A | %B |
|---|---|---|---|
| | 0 | 75 | 25 |
| | 45 | 25 | 75 |
| | 45.1 | 5 | 95 |
| | 46.1 | 5 | 95 |
| | 46.2 | 75 | 25 |

The mobile phase starts as 75% eluent A / 25% eluent B at time zero, then the composition is modified gradually and linearly such that after 45 minutes the mobile phase comprises 25% eluent A and 75% eluent B. A steeper linear gradient is then applied such that after 45.1 minutes the mobile phase comprises 5% eluent A and 95% eluent B. This composition is held until 46.1 minutes then is modified to 75% eluent A and 25% eluent B by 46.2 minutes. Upon completion of data collection at 33 minutes, the eluent composition is held at 75% eluent A / 25% eluent B up to 52 minutes order to re-equilibrate the column.

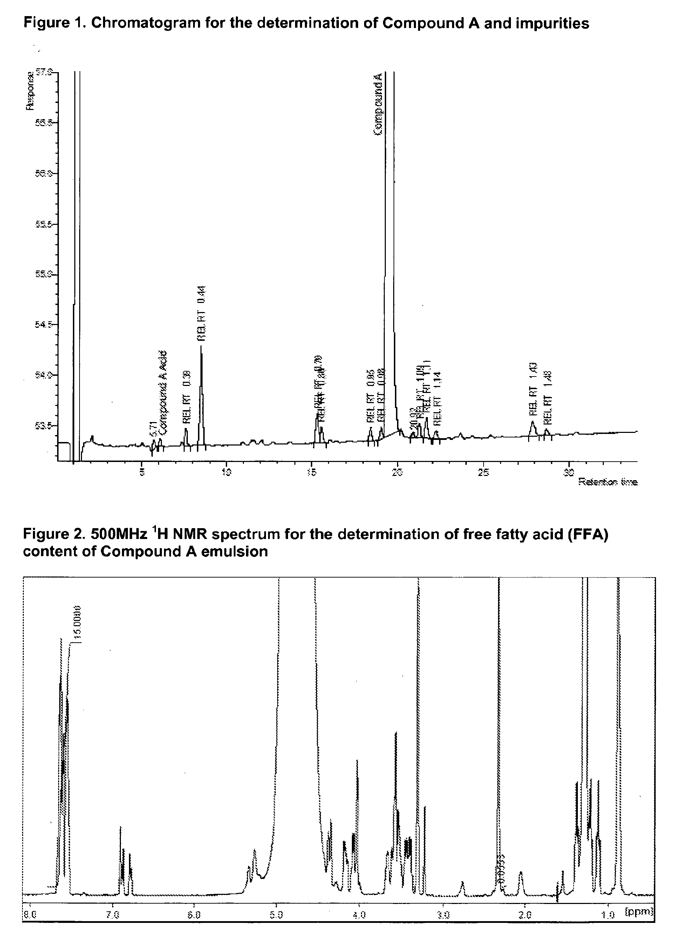

Separation of impurities was performed using a column 10 cm long x 4.6 mm internal diameter packed with Waters Symmetry C18 stationary phase having 3.5µm particle size. The mobile phase flow rate was 1.0 mL/minute, temperature was controlled at 30°C, and impurity concentration was determined by comparison of absorbance at 280nm, measured using a variable wavelength uv detector, with that of an external reference standard solution comprising 1.5 mg/mL Compound A in acetonitrile. A typical sample chromatogram is shown in Figure 1.

The major impurity detected was a hydrolytic degradation product which is an inactive metabolite of Compound A and has the structure: 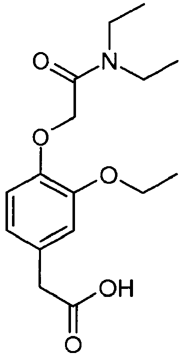 ([3-ethoxy-4-[(*N,N-*diethylcarbamido)methoxy]phenyl]acetic acid, hereinafter referred to as A-acid).

### pH by potentiometric determination

pH was measured using a combination electrode calibrated using pH 4.01 and pH 9.21 buffers. The electrode was rinsed with methanol followed by water between measurements.

### Osmolality

Osmolality was determined by depression of freezing point, using a Roebling Osmometer (Hermann Roebling, Berlin, Germany) calibrated using purified water and an aqueous 300 mOsmol/kg standard.

### Droplet size by Photon Correlation Spectroscopy

Photon correlation spectroscopy (PCS) was used to determine z-average droplet diameters and polydispersity indices for emulsions. A Brookhaven Instruments BI-9000 was used to collect data over a period of 10 minutes at a detection angle of 90°. Measurements were made at ambient temperature. Vials of emulsion were gently inverted once before measurements were taken (no air bubbles were introduced). A small quantity of emulsion was placed in a sample tube containing diluent (a particle-free saturated solution of Compound A in water) and inverted several times until homogenous in appearance.

### Volume mean diameter

Volume mean diameter was determined by differential centrifugal sedimentation using a CPS Disc Centrifuge Model DC2400. With the disc spinning at 24,000rpm, the disc density gradient was made up by sequential injection through a standard injection port of equal 1.6mL aliquots of 16, 15, 14, 13, 12, 11, 10, 9, and 8%w/w sucrose in D₂O, followed by 0.5mL dodecane. The disc was allowed to equilibrate for 25 minutes, during which time a low density disc injection port was fitted. The particle size distribution of Compound A emulsion was determined using a sample concentration of 30µL emulsion / 1mL 20%w/w sucrose in D₂O, against an external calibration standard comprising 0.4µm polypropylene.

### Large globule content (% of droplets > 5µm, "PFAT5")

Large globule content was determined in accordance with USP <729> by light obscuration using an AccuSizer 780-APS Optical Particle Sizer (Particle Sizing Systems, USA), which employs the technique of single particle optical sensing (SPOS). Measurements were performed on undiluted samples of Compound A emulsion using extinction mode which detects particles in the range 1.8 to 400µm. Results were reported as the volume weighted percentage of fat greater than 5µm ("PFAT5") and / or as mean # particles > 4.99µm / mL.

### Free fatty acid (FFA) content by ¹H Nuclear Magnetic Resonance (NMR) spectrometry

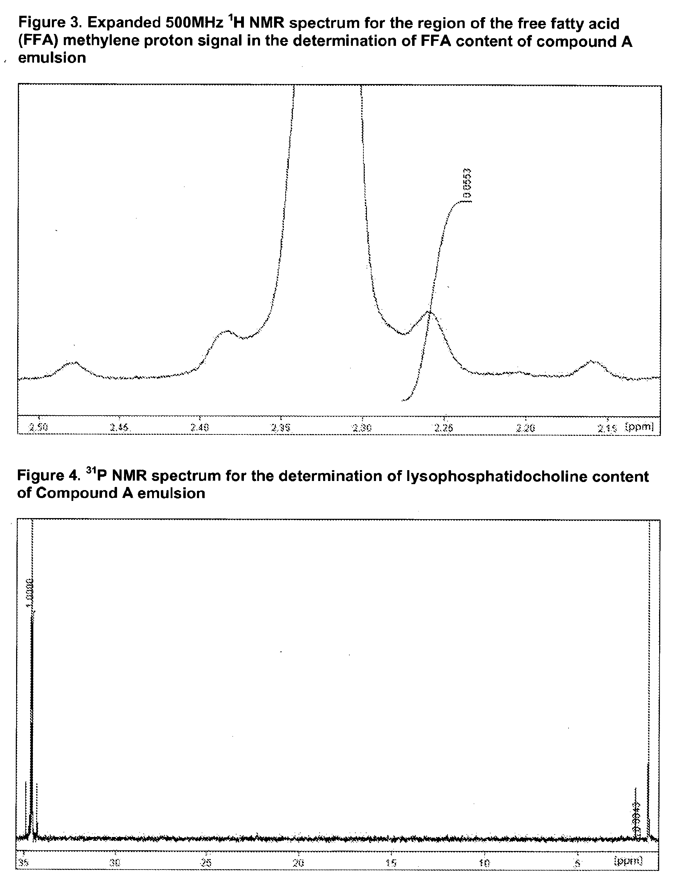

FFA content was determined using 1H NMR by comparison of the FFA methylene proton signal with the proton signal from an internal standard comprising triphenylphosphineoxide (TPPO). Approximately 100mg of Compound A emulsion and 5mg of TPPO were weighed accurately into a vial and diluted with 1.0mL of D4 methanol. A homonuclear decoupled ¹H NMR spectrum (≥400 MHz) was obtained using a 90° pulse at a temperature of 300°K. The chemical shift of the D4 methanol multiplet was set to 3.30ppm and the TPPO proton signal at 7.3-7.6ppm and the FFA methylene proton signal at 2.21 - 2.26ppm were integrated accurately. A typical ¹H NMR spectrum is shown in Figure 2 and an expanded ¹H NMR spectrum in Figure 3.

### Lysophosphatidylcholine content by ³¹P NMR

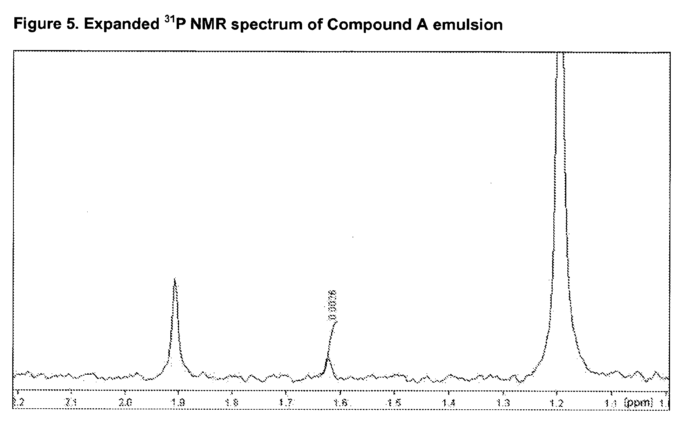

Lysophosphatidylcholine (LysoPC) content was determined using 1H NMR by comparison of the LysoPC ³¹P signal with the ³¹P signal from an external standard comprising triphenylphosphineoxide (TPPO). Samples were prepared as described above for FFA content. A proton decoupled ³¹P NMR spectrum (≥162 MHz) was obtained using a 30° pulse at a temperature of 300°K. The chemical shift of the TPPO resonance was set to 34.6ppm and this resonance and the LysoPC resonance at approximately 1.7ppm were integrated accurately. A typical ³¹P NMR spectrum is shown in Figure 4 and an expanded ³¹P NMR spectrum in Figure 5.

The materials listed in the table below were used in the examples which follow:

| **Material** | **Pharmacopoeial Name** | **Supplier** | **Function** |
|---|---|---|---|
| Soybean oil | Soya bean oil | Lipoid | Solvent |
| Miglyol 810N | Medium chain triglyceride | Sassol | Solvent |
| Labrafac WL1349 | Medium chain triglyceride | Gattefosse | Solvent |
| Lipoid E80 | Egg-derived lecithin | Lipoid | Emulsifier |
| Glycerol | Glycerol | Sigma-Aldrich | Tonicity modifier |
| Oleic acid | Oleic acid | Sigma-Aldrich | Stabilizer |
| L-Histidine | L-Histidine | Sigma-Aldrich | Buffer |
| EDTA | Ethylenediaminetetraacetic acid (as disodium salt) | Sigma-Aldrich | Antimicrobial agent |
| WFI | Water for injection | AstraZeneca | Solvent |
| NaOH | Sodium hydroxide | Sigma-Aldrich | modifier |
| Lipoid S75 | Soy-derived lecithin | Lipoid | Emulsifier |
| Lipoid MCT | Medium chain tryglyceride | Lipoid | Solvent |
| Solutol HS 15 | Macrogol HS15 | BASF | Emulsifier |
| Poloxamer 188 | Poloxamer 188 | BASF | Emulsifer |
| Disodium hydrogen orthophosphate anhydrous | Disodium hydrogen orthophosphate anhydrous | Sigma-Aldrich | Buffer |
| Citric acid monohydrate | Citric acid monohydrate | Sigma-Aldrich | Buffer |

### Example 1: Evaluation of preliminary Compound A emulsions (WO2005/009420)

Compound A emulsions were prepared as defined in Example 6 of WO2005/009420 with the following compositions (all values % w/w).

| | **Batch 1** | **Batch 2** | **Batch 3** | **Batch 4** | **Batch 5** | **Batch 6** |
|---|---|---|---|---|---|---|
| Batch size | 3.5L | 3.5L | 2.0L | 0.5L | 0.5L | 0.5L |
| Soybean oil | 20 | 20 | 20 | 5 | 4 | 4 |
| Miglyol 810N | 0 | 0 | 0 | 5 | 0 | 3 |
| Labrafac WL1349 | 0 | 0 | 0 | 0 | 2.5 | 0 |
| Compound A | 4 | 4 | 0 | 8 | 10 | 20 |
| Egg lecithin Lipoid E80 | 2.4 | 2.4 | 2.4 | 1.2 | 1.2 | 1.2 |
| Glycerol | 2.5 | 2.5 | 2.5 | 2.25 | 2.25 | 2.25 |
| Oleic acid | 0.03 | 0.03 | 0.03 | 0.03 | 0 | 0.3 |
| Histidine | 0.1 | 0.1 | 0.1 | 0.1 | 0.1 | 0.1 |
| EDTA | 0.005 | 0.005 | 0.005 | 0.005 | 0.005 | 0.005 |
| WFI | To 100 | To 100 | To 100 | To 100 | To 100 | To 100 |
| NaOH | To pH 8 | To pH 8 | To pH 8 | To pH 8 | To pH 8 | To pH 8 |

Evaluation was performed after storage under refrigerated conditions (2-8°C) for approximately 10 months (Batches 1-3) or approximately 6 months (batches 4-6).

| **Emulsion Batch** | **Appearance** | **Mean Effective Diameter ¹ (nm)** | **Mean Polydispersity Index¹** | **Mean # particles > 4.99µm / mL** |
|---|---|---|---|---|
| Batch 1 | No oiling | > 1µm | - | 3.22x10⁶ |
| Batch 2 | Large oil droplets visible | > 1µm | - | 2.22x10⁷ |
| Batch 3 | Surface oiling | > 1µm | - | 1.17x10⁶ |
| Batch 4 | Surface oiling | 274 | 0.357 | 8.05x10⁵ |
| Batch 5 | No oiling | > 1 µm | - | 1.94x10⁷ |
| Batch 6 | Oily droplets | 173 | 0.157 | 1.15x10⁶ |

| | | | | |
|---|---|---|---|---|
| ¹ Determined by Photon Correlation Spectroscopy | | | | |

The samples were found to have undergone extensive physical degradation including visible surface oiling and coalescence. Even for the two samples which exhibitec mean effective diameter in line with the values presented in WO2005/009420, the Large Globule Count (mean # particles > 4.99µm / mL) was unacceptably high. On this basis, it was concluded that the pharmaceutical presentations described in WO2005/009420 were not suitable for clinical use.

### Example 2: Production of an improved Compound A emulsion at 100g scale

The pharmaceutical formulation made in the example below comprises the following components:

| **Component** | **Purpose** | **Weight%** |
|---|---|---|
| Compound A | Active ingredient | 6 |
| Lipoid MCT (PhEur) | Oil | 9 |
| Oleic acid Ph Eur | Stabilizer | 0.3 |
| Lipoid S75 Lecithin | Emulsifier | 2.5 |
| Glycerol | Tonicity | 2 |
| Water for injection | | To 100% |

### Emulsion adjusted to pH 7 with 1 M NaOH.

Compound A, the oil and oleic acid were weighed into a 150 ml tall form beaker (to produce the oil phase). The beaker was then swirled by hand until the oil phase became homogenous in appearance.

The lecithin, glycerol and water were weighed into a second 150 ml tall form beaker (to produce the aqueous phase). The aqueous phase ingredients were then dispersed using an Ultra Turrax T25 homogenizer at 11,000 rpm for 1 minute.

The homogenizer head was then transferred to the oil phase beaker and the aqueous phase ingredients were added and homogenized at 11,000 rpm for 1 minute. This produced a coarse emulsion premix.

The emulsion premix was then introduced to the Microfluidizer 120E (previously primed with water) and operated at approximately 14,000 psi. The output was run to waste until it became completely cloudy and then collected in a clean beaker and processed for a further five Microfluidizer cycles before being collected in a suitable glass bottle.

The emulsion was allowed to cool to ambient temperature in the bottle before the pH was adjusted to 7 with 1 M sodium hydroxide solution.

The emulsion was passed through a syringe filter (Millipore Express PES membrane, 0.22 µm pore size, ref no. SLGP033RS), before being filled into 10 ml type 1 glass vials and overlaid with nitrogen.

The analytical data generated on the emulsion are summarized in the table below:

| | |
|---|---|
| Compound A (mg/ml) | 55.7 |
| Final pH | 7.04 |
| Osmolality (mOsmoles/kg H₂O) | 279 |
| Mean droplet size (nm)¹ | 145 |
| Mean polydispersity index¹ | 0.089 |
| % droplets > 5µm in diameter (by mass) | 0.005 |

| | |
|---|---|
| ¹ Determined by Photon Correlation Spectroscopy | |

The results above demonstrate that the emulsion oil droplets are very small, permitting sterilization by filtration and falling well within the size limits specified in USP <729>, indicating suitability for intravenous administration.

### Example 3: Production of an improved Compound A emulsion at 200g scale The pharmaceutical formulation made in the example below comprises the following components:

| **Component** | **Purpose** | **Weight%** |
|---|---|---|
| Compound A | Active ingredient | 6 |
| Lipoid MCT (PhEur) | Oil | 9 |
| Oleic acid Ph Eur | Stabilizer | 0.03 |
| Lipoid S75 Lecithin | Emulsifier | 2.5 |
| Glycerol | Tonicity | 2.25 |
| Water for injection | | To 100% |

Emulsion adjusted to pH 7 with 1 M NaOH.

Compound A, the oil and oleic acid were weighed into a 250 ml tall form beaker (to produce the oil phase). The beaker was then swirled by hand until the oil phase became homogenous in appearance.

The lecithin, glycerol and water were weighed into a second 250 ml tall form beaker (to produce the aqueous phase). The aqueous phase ingredients were then dispersed using an Ultra Turrax T25 homogenizer at 11,000 rpm for 2 minutes.

The homogenizer head was then transferred to oil phase beaker and the aqueous phase ingredients were added and homogenized at 11,000 rpm for 2 minutes. This produced a coarse emulsion premix.

The emulsion premix was then introduced to the Microfluidizer 120E (previously primed with water) and operated at approximately 14,000 psi. The output was run to waste until it became completely cloudy and then collected in a clean beaker and processed for a further 5 Microfluidizer cycles before being collected in a suitable glass bottle.

The emulsion was allowed to cool to ambient temperature in the bottle before the pH was adjusted to 7 with 1 M sodium hydroxide solution.

The emulsion was passed through a syringe filter (Millipore Express PES membrane, 0.22 µm pore size, ref no. SLGP033RS), before being filled into 5 ml type 1 glass vials and overlaid with nitrogen.

The analytical data generated on the emulsion are summarized in the table below:

| | |
|---|---|
| Compound A (mg/ml) | 55.9 |
| Final pH | 6.84 |
| Osmolality (mOsmoles/kg H₂O) | 323 |
| % droplets > 5µm in diameter (by mass) | 0.002 |

The results above demonstrate that the emulsion oil droplets are very small, permitting sterilization by filtration and falling well within the limit for droplets larger than 5 µm specified in USP <729>, indicating suitability for intravenous administration.

### Example 4: Production of an improved Compound A emulsion at 2kg scale

The pharmaceutical formulation made in the example below comprises the following components:

| **Component** | **Purpose** | **Weight%** |
|---|---|---|
| Compound A | Active ingredient | 6 |
| Lipoid MCT (PhEur) | Oil | 9 |
| Oleic acid Ph Eur | Stabilizer | 0.03 |
| Lipoid S75 Lecithin | Emulsifier | 2.5 |
| Glycerol | Tonicity | 2.25 |
| Water for injection | | To 100% |

Emulsion adjusted to pH 7 with 1 M NaOH.

Compound A, the oil and oleic acid were weighed into a 3 L tall form beaker (to produce the oil phase). The beaker was then swirled by hand until the oil phase became homogenous in appearance.

The lecithin, glycerol and water were weighed into a second 3 L tall form beaker (to produce the aqueous phase). The aqueous phase ingredients were then dispersed using an Ultra Turrax T25 homogenizer at 22,000 rpm for 10 minutes.

The aqueous phase was then added to the oil phase under mixing at 11,000 rpm and then a coarse emulsion premix was produced by mixing at 22,000 rpm for 10 minutes. The emulsion premix was then introduced to the Microfluidizer 110F (previously primed with water) and operated at approximately 7,000 to 14,000 psi. The output was run to waste until it became completely cloudy and then collected in a clean beaker and processed for a further 5 Microfluidizer cycles before being collected in a suitable glass bottle.

The emulsion was allowed to cool to ambient temperature in the bottle before the pH was adjusted to 7 with 1 M sodium hydroxide solution.

The emulsion was passed through a filter comprising a 1.2 µm pore diameter (Pall Kleenpak, polypropylene membrane) followed by a 0.2 µm pore size sterilizing grade filter (Sartorius Sartobran P 500, cellulose acetate membrane) before being filled into 10 ml type 1 glass vials and overlaid with nitrogen.

Analytical data were generated on the emulsion during storage at 5°C over a period of nine months and are summarized in the table below. The data demonstrate that the emulsion had sufficient stability to allow a nine month shelf-life at 5°C to be assigned.

### Stability data for improved Compound A emulsion, stored at 5°C

| **Test** | **Specification** | **Initial** | **4 weeks** | **13 weeks** | **28 weeks** | **39 weeks** |
|---|---|---|---|---|---|---|
| Description | A white to pale yellow homogeneous emulsion practically free from extraneous matter and large oil droplets | A white homogeneous emulsion practically free from extraneous matter and large oil droplets | NCH | NCH | NCH | NCH |
| Identity by HPLC | Retention time consistent with that of reference standard | Retention time consistent with that of reference standard | NCH | NCH | NCH | NCH |
| Assay (mg/ml) | 54-66 | 61.9 mg/ml | 61.1 mg/ml | 61.1 mg/ml | 60.8 mg/ml | 60.3% w/w |
| Largest single impurity | 0.5% w/w maximum | 0.29% w/w | 0.28% w/w | 0.28% w/w | 0.28% w/w | 0.28% w/w |
| Total Impurities^{a} | 2.0% w/w maximum | 0.68% w/w | 0.61% w/w | 0.66% w/w | 0.70% w/w | 0.82% w/w |
| pH | 4.5-8.0 | 6.6 | 6.2 | 6.0 | 5.5 | 5.0 |
| Large Droplet Count (% > 5µm <0.05%) | <0.05% | 0.001% | 0.001% | 0.002% | 0.004% | 0.002% |
| Osmolality | Non spec test | 340 | 346 | 331 | 335 | 341 |
| Sterility | Complies with EP | Complies | NT | NT | NT | NT |

| | | | | | | |
|---|---|---|---|---|---|---|
| ^{a} The figures in parentheses represent the number of organic impurities ≥0.05% w/w NCH No Change NT Not Tested | | | | | | |

### Example 5: Production of an improved Compound A emulsion at 2kg scale

The pharmaceutical formulation made in the example below comprises the following components:

| **Component** | **Purpose** | **Weight%** |
|---|---|---|
| Compound A | Active ingredient | 6 |
| Lipoid MCT (PhEur) | Oil | 9 |
| Histidine Ph Eur | Buffer | 0.1 |
| Lipoid S75 Lecithin | Emulsifier | 1.25 |
| Glycerol | Tonicity | 2.25 |
| Water for injection | | To 100% |

Emulsion adjusted to pH 7 with 1 M NaOH.

Compound A, the oil and histidine were weighed into a 3 L tall form beaker (to produce the oil phase). The beaker was then swirled by hand until the oil phase became homogenous in appearance.

The lecithin, glycerol and water were weighed into a second 3 L tall form beaker (to produce the aqueous phase). The aqueous phase ingredients were then dispersed using an Ultra Turrax T25 homogenizer at 22,000 rpm for 10 minutes.

The aqueous phase was then added to the oil phase under mixing at 11,000 rpm and then a coarse emulsion premix was produced by mixing at 22,000 rpm for 10 minutes.

The emulsion premix was then introduced to the Microfluidizer 110F (previously primed with water) and operated at approximately 7,000 to 14,000 psi. The output was run to waste until it became completely cloudy and then collected in a clean beaker and processed for a further 5 Microfluidizer cycles before being collected in a suitable glass bottle.

The emulsion was allowed to cool to ambient temperature in the bottle before the pH was adjusted to 7 with 1 M sodium hydroxide solution if required.

The emulsion was passed through a filter comprising a 1.2 µm pore diameter (Pall Kleenpak, polypropylene membrane) followed by a 0.2 µm pore size sterilizing grade filter (Sartorius Sartopore 2 500, poly ether sulphone membrane) before being filled into 6 ml type 1 glass vials and overlaid with nitrogen.

The analytical data generated on the emulsion are summarized in the table below:

| | |
|---|---|
| Compound A (mg/ml) | 59.3 |
| Final pH | 7.3 |
| Osmolality (mOsmoles/kg H₂O) | 351 |
| Mean droplet size (nm) | 157 |
| % droplets > 5µm in diameter (by mass) | 0.025 |

### Example 6: Production of an improved Compound A emulsion at 2kg scale

The pharmaceutical formulation made in the example below comprises the following components:

| **Component** | **Purpose** | **Weight%** |
|---|---|---|
| Compound A | Active ingredient | 10 |
| Lipoid MCT (PhEur) | Oil | 5 |
| Macrogol HS 15 | Emulsifier | 1.6 |
| Poloxamer 188 | Emulsifier | 2.4 |
| Citrate buffer pH 5 | Buffer | To 100% |

The buffer in the example below comprises the following components:

| **Component** | **Weight%** |
|---|---|
| Disodium Hydrogen Orthophosphate Anhydrous | 1.46 |
| Citric Acid Monohydrate | 1.02 |
| Water for Injection | To 100% |

The buffer was prepared by dissolving the appropriate quantities of Disodium Hydrogen Orthophosphate Anhydrous and Citric Acid Monohydrate in Water for Injection. Compound A and the oil were weighed into a beaker and mixed until homogenous (to produce the oil phase). Macrogol HS15 was dissolved in buffer then the Poloxamer 188 added slowly under vigorous stirring. Stirring was continued until the Poloxamer 188 was dissolved (to produce the aqueous phase).

The aqueous phase was then added to the oil phase under mixing at 11,000 rpm and then a coarse emulsion premix was produced by mixing at 22,000 rpm for 10 minutes. The emulsion premix was then introduced to the Microfluidizer 110F (previously primed with water) and operated at approximately 7,000 to 14,000 psi. The output was run to waste until it became completely cloudy and then collected in a clean beaker and processed for a further 5 Microfluidizer cycles before being collected in a suitable glass bottle.

The emulsion was allowed to cool to ambient temperature in the bottle.

The emulsion was passed through filters comprising a 1.2 µm pore diameter (Pall Kleenpak, polypropylene membrane) followed by a 0.2 µm pore size sterilizing grade filter (Sartorius Sartopore 2 300, polyether sulphone membrane) before being filled into 10 ml type 1 glass vials and overlaid with nitrogen.

The analytical data generated on the emulsion are summarized in the table below:

| | |
|---|---|
| Compound A (mg/ml) | 98.1 |
| Final pH | 5.1 |
| Osmolality (mOsmoles/kg H₂O) | 386 |
| Mean droplet size (nm) | 160 |
| % droplets > 5µm in diameter (by mass) | 0.044 |

The results above demonstrate that the emulsion oil droplets are very small, permitting sterilization by filtration, and falling well within the limit for droplets larger than 5 µm specified in USP <729>, indicating suitability for intravenous administration.

Various modifications of the invention, in addition to those described herein, will be apparent to those skilled in the art from the foregoing description. Such modifications are also intended to fall within the scope of the appended claims. Each reference (including, but not limited to, journal articles, U.S. and non-U.S. patents, patent application publications, international patent application publications, and the like) cited in the present application is incorporated herein by reference in its entirety.

### Example 7: Production and sterilisation of an optimised Compound A emulsion at 3Kg scale

The pharmaceutical formulation made in the example below comprises the following components:

| **Component** | **Purpose** | **Amount (% weight / weight)** |
|---|---|---|
| Compound A | Active ingredient | 6 |
| Lipoid MCT (PhEur) | Oil | 9 |
| Soy-derived lecithin (Lipoid S75) | Emulsifier | 1.1 |
| L-Histidine | Buffering agent | 0.5 |
| Disodium edetate | Antimicrobial agent | 0.0025 |
| Glycerol | Tonicity adjuster | 1.75 |
| Water for injection | Solvent | To 100 |

The required quantities of Compound A and the medium chain triglyceride oil (Lipoid MCT PhEur) were weighed into a beaker (400mL standard form) then swirled to form a homogeneous mixture (the oil phase).

The L-histidine and disodium edetate were weighed into a second beaker (3000mL tall form), the water for injection was added and the mixture was stirred to dissolve using a magnetic stirrer (20 minutes approx.). The magnetic stirrer was removed, the soy-derived lecithin (Lipoid S75) and glycerol were added, and the resulting mixture was homogenized using an Ultra Turrax T25 with S25N-18G head (22,000 rpm, 10 minutes approx.) to form the aqueous phase.

The oil phase was added to the aqueous phase with continuous slow homogenization (Ultra Turrax, 11,000rpm, 20 seconds approx.); after completion of transfer, homogenization was continued (Ultra Turrax, 22,000rpm, 10 minutes) to produce a coarse emulsion.

The coarse emulsion was transferred to the hopper of a M-110EH-30 microfluidiser configured with a diamond-coated interaction chamber with 75µm channel diameter. Microfluidisation was performed over 8 passes using a peak pressure of 14000psi with continuous cooling to a target temperature of 20°C; the first 200mL of emulsion from the first pass was discarded and a clean collection vessel was used after the first and final pass.

The resulting emulsion was filtered through a 1.2µm filter (Pall KA1J012P2) using a Watson Marlow 505 peristaltic pump at 50 rpm.

A sub-batch of sterile vials was produced as follows. Approximately 1 litre was taken from the mother batch and filled under aseptic conditions into vials (neutral type 1 glass sealed with ethylene tetrafluoroethylene [ETFE] coated rubber stoppers with aluminium and plastic crimp, 5mL fill volume with nitrogen overlay). These vials were sterilized by autoclave using a standard pharmacopoeial cycle (121°C/15 minutes). This batch was designated Batch 1/1.

The remainder of the mother batch was sterilized by filtration under air (15psi) using a 0.2µm filter (4 Sartopore 2 500 filters operating in parallel) then filled under aseptic conditions into vials as described above for Batch 1/1. Half of the vials so produced were designated Batch 1/2; the remainder were subjected to further sterilization by autoclave as described above for Batch 1/1. This batch was designated Batch 1/3.

A second mother batch was produced using the process described above but at reduced scale (2kg) and with the addition of nitrogen sparging during manufacture of the aqueous phase, coarse and fine emulsion.

A sub-batch of sterile vials was produced as follows. Approximately 1 litre was taken from the mother batch and filled under aseptic conditions into vials as described above for Batch 1/1. These vials were sterilized by autoclave using a standard pharmacopoeial cycle (121°C / 15 minutes). This batch was designated Batch 2/1.

The remainder of the second mother batch was sterilized by filtration under nitrogen (15psi) using a 0.2µm filter (4 Sartopore 2 500 filters operating in parallel) then filled under aseptic conditions into vials as described above for Batch 1/1. This batch was designated Batch 2/2.

The stability of these sub-batches was evaluated as follows. Samples from Batch 1 were stored under refrigeration (5°C), long-term conditions (25°C / 60% relative humidity), accelerated conditions (40°C / 75% relative humidity) and stressed conditions (50°C / ambient humidity. Samples from Batch 2 were stored under accelerated conditions (40°C / 75% relative humidity) and stressed conditions (50°C / ambient humidity only. Testing was performed prior to set-down, after 1 month and, after 3 months. The results are presented in Tables 1 to 5 below.

The initial results indicate that sparging during processing had little impact upon imputiry levels. The optimized formulation was superior to the preliminary formulation described in WO2005/009420, as shown in particular by the large globule count which was reduced by one to two orders of magnitude. Sterilisation by autoclave was observed to result in a minor increase in impurity levels and an increase in the concentration of large globules (PFAT5) within the USP <729> limit of 0.05%. These results demonstrated that the optimized formulation was suitable for sterilization by filtration, autoclave or a combination of these two processes.

**Table 1 Stability study for Example 7 Batch 1/1 (un-sparged, sterilised by autoclave)**

| **Storage condition** | **None** | **5°C** | **25°C / 60% Relative Humidity** | **40°C / 75% Relative Humidity** | **50°C / Ambient Humidity** |
|---|---|---|---|---|---|
| **Time**-**point** | Initial | 1 month | 1 month | 1 month | 1 month |
| **Description** | Complies | NCH | NCH | NCH | NCH |
| **Compound A (%w/w)** | 61.3 | 60.7 | 60.7 | 60.4 | 60.1 |
| **A-acid (%)** | 0.13 | 0.25 | 0.50 | 0.88 | 1.32 |
| **Unknown impurities (%)** | 0.10 | 0.05 | 0.05 | 0.10 | 0.11 |
| **Total Impurities (%)** | 0.23 | 0.30 | 0.55 | 0.98 | 1.43 |
| **Lyso-PC (%w/w)** | N/D | N/D | N/D | N/D | N/D |
| **FFA (mMol/kg)** | 2.7 | 3.2 | 2.7 | 2.8 | 2.3 |
| **Osmolality (mOsmol/Kg)** | 352 | NT | NT | NT | NT |
| **pH** | 7.6 | 7.5 | 7.4 | 7.2 | 7.0 |
| **Mean particle size (nm)** | 137.1 | 138.3 | 138.6 | 138.7 | 146.5 |
| **PFAT5 (%)** | 0.034 | 0.018 | 0.016 | 0.045 | 0.068 |
| **Mean # particles > 4.99µm / mL** | 7.04x10⁴ | 2.95x10⁴ | 3.38x10⁴ | 1.36x10⁵ | 3.17x10⁵ |
| **Edetate (%w/w)** | 0.001 | NT | NT | NT | NT |

| | | | | | |
|---|---|---|---|---|---|
| **Key:** Complies = A white to pale yellow homogeneous emulsion practically free from extraneous particulate matter and visible oil droplets. NCH = No change N/D = Not detected NT = Not tested | | | | | |

**Table 2 Stability Study for Example 7 Batch 1/2 (Un-sparged, Sterilised by Filtration)**

| **Storage condition** | **None** | **5°C** | **25°C** / **60% Relative Humidity** | **40°C / 75% Relative Humidity** | **50°C / Ambient Humidity** |
|---|---|---|---|---|---|
| **Time**-**point** | Initial | 1 month | 1 month | 1 month | 1 month |
| **Description** | Complies | NCH | NCH | NCH | NCH |
| **Compound A (%w**/**w**) | 54.4 | 54.3 | 54.3 | 54.0 | 53.6 |
| **A-acid (%)** | 0.05 | 0.20 | 0.46 | 0.86 | 1.29 |
| **Unknown impurities (%)** | 0.05 | <0.05 | <0.05 | <0.05 | 0.05 |
| **Total Impurities (%)** | 0.10 | 0.20 | 0.46 | 0.86 | 1.34 |
| **Lyso-PC (%w/w)** | N/D | N/D | N/D | N/D | N/D |
| **FFA (mMol/kg)** | 2.8 | 2.6 | 2.8 | 2.4 | 2.5 |
| **Osmolality (mOsmol/Kg)** | 317 | NT | NT | NT | NT |
| **pH** | 7.7 | 7.6 | 7.4 | 7.2 | 7.1 |
| **Mean particle size (nm)** | 134.8 | 132.7 | 135.7 | 132.9 | 138.1 |
| **PFAT5 (%)** | 0.006 | 0.007 | 0.008 | 0.006 | 0.015 |
| **Mean # particles > 4.99µm / mL** | 3.84x10⁴ | 3.19x10⁴ | 4.80x10⁴ | 3.55x10⁴ | 7.67x10⁴ |
| **Edetate (%w**/**w**) | 0.001 | NT | NT | NT | NT |

| | | | | | |
|---|---|---|---|---|---|
| **Key:** Complies = A white to pale yellow homogeneous emulsion practically free from extraneous particulate matter and visible oil droplets. NCH = No change N/D = Not detected NT = Not tested | | | | | |

**Table 3 Stability study for Example 7 Batch 1/3 (un-sparged, sterilised by filtration and autoclave)**

| **Storage condition** | **None** | **5°C** | **25°C** / **60% Relative Humidity** | **40°C / 75% Relative Humidity** | **50°C** / **Ambient Humidity** |
|---|---|---|---|---|---|
| **Time**-**point** | Initial | 1 month | 1 month | 1 month | 1 month |
| **Description** | Complies | NCH | NCH | NCH | NCH |
| **Compound A (%w/w)** | 54.6 | 54.2 | 54.2 | 53.7 | 53.7 |
| **A-acid (%)** | 0.14 | 0.28 | 0.54 | 0.89 | 1.35 |
| **Unknown impurities (%)** | 0.05 | 0.05 | 0.05 | 0.10 | 0.20 |
| **Total Impurities (%)** | 0.19 | 0.33 | 0.59 | 0.99 | 1.55 |
| **Lyso-PC (%w/w)** | N/D | N/D | N/D | N/D | N/D |
| **FFA (mMol/kg)** | 2.1 | 2.5 | 2.5 | 2.1 | 2.9 |
| **Osmolality (mOsmol/Kg)** | 324 | NT | NT | NT | NT |
| **pH** | 7.6 | 7.5 | 7.4 | 7.2 | 7.1 |
| **Mean particle size (nm)** | 133.7 | 136.7 | 135.6 | 138.1 | 138.7 |
| **PFAT5 (%)** | 0.020 | 0.031 | 0.023 | 0.028 | 0.031 |
| **Mean # particles > 4.99µm / mL** | 6.31x10⁴ | 1.14x10⁵ | 9.34x10⁴ | 1.12x10⁵ | 1.33x10⁵ |
| **Edetate (%w**/**w**) | 0.001 | NT | NT | NT | NT |

| | | | | | |
|---|---|---|---|---|---|
| **Key:** Complies = A white to pale yellow homogeneous emulsion practically free from extraneous particulate matter and visible oil droplets. NCH = No change N/D = Not detected NT = Not tested | | | | | |

**Table 4 Stability study for Example 7 Batch 2/1 (sparged, sterilised by autoclave)**

| **Storage condition** | **None** | **5°C** | **25°C** / **60% Relative Humidity** | **40°C / 75% Relative Humidity** | **50°C** / **Ambient Humidity** |
|---|---|---|---|---|---|
| **Time-point** | Initial | 1 month | 1 month | 1 month | 1 month |
| **Description** | Complies | NT | NT | NCH | NCH |
| **Compound A (%w/w)** | 62.1 | NT | NT | 61.0 | 60.5 |
| **A-acid (%)** | 0.11 | NT | NT | 0.92 | 1.34 |
| **Unknown impurities (%)** | 0.15 | NT | NT | 0.16 | 0.16 |
| **Total Impurities (%)** | 0.26 | NT | NT | 1.08 | 1.50 |
| **Lyso-PC (%w/w)** | N/D | NT | NT | N/D | N/D |
| **FFA (mMol/kg)** | 2.4 | NT | NT | 2.4 | 2.7 |
| **Osmolality (mOsmol/Kg)** | 355 | NT | NT | NT | NT |
| **pH** | 7.6 | NT | NT | 7.2 | 7.0 |
| **Mean particle size (nm)** | 137.1 | NT | NT | 138.9 | 140.9 |
| **PFAT5 (%)** | 0.026 | NT | NT | 0.028 | 0.022 |
| **Mean # particles > 4.99µm / mL** | 6.02x10⁴ | NT | NT | 1.09x10⁵ | 9.72x10⁴ |
| **Edetate (%w/w)** | 0.002 | NT | NT | NT | NT |

| | | | | | |
|---|---|---|---|---|---|
| **Key:** Complies = A white to pale yellow homogeneous emulsion practically free from extraneous particulate matter and visible oil droplets. NCH = No change N/D = Not detected NT = Not tested | | | | | |

**Table 5 Stability study for Example 7 Batch 2/2 (sparged, sterilised by filtration)**

| **Storage condition** | **None** | **5°C** | **25°C / 60% Relative Humidity** | **40°C / 75% Relative Humidity** | **50°C / Ambient Humidity** |
|---|---|---|---|---|---|
| **Time**-**point** | Initial | 1 month | 1 month | 1 month | 1 month |
| **Description** | Complies | NT | NT | NCH | NCH |
| **Compound A (%w/w)** | 58.3 | NT | NT | 57.6 | 57.3 |
| **A-acid (%)** | <0.05 | NT | NT | 0.86 | 1.30 |
| **Unknown impurities (%)** | 0.10 | NT | NT | 0.15 | 0.16 |
| **Total Impurities (%)** | 0.10 | NT | NT | 1.01 | 1.46 |
| **Lyso-PC (%w**/**w**) | N/D | NT | NT | N/D | N/D |
| **FFA (mMol/kg)** | 2.5 | NT | NT | 2.6 | 3.2 |
| **Osmolality (mOsmol/Kg)** | 341 | NT | NT | NT | NT |
| **pH** | 7.7 | NT | NT | 7.2 | 7.0 |
| **Mean particle size (nm)** | 133.1 | NT | NT | 134.6 | 134.8 |
| **PFAT5 (%)** | 0.003 | NT | NT | 0.010 | 0.019 |
| **Mean # particles > 4.99µm / mL** | 2.26x10⁴ | NT | NT | 7.04x10⁴ | 1.47x10⁵ |
| **Edetate (%w**/**w**) | 0.002 | NT | NT | NT | NT |

| | | | | | |
|---|---|---|---|---|---|
| **Key:** Complies = A white to pale yellow homogeneous emulsion practically free from extraneous particulate matter and visible oil droplets. NCH = No change N/D = Not detected NT = Not tested | | | | | |

## Claims

1. An injectable emulsion comprising:
a substituted phenylacetic acid ester compound which is [3-ethoxy-4-[(*N,N-*diethylcarbamido)-methoxy]phenyl]acetic acid *n*-propyl ester;
a medium chain triglygeride present from 5 wt% to 15 wt% ;
an emulsifier which is soybean-derived lecithin;
a tonicity modifier; and
water;
and optionally further comprising one or more of the following selected from a pH buffering agent, a stabilizer, and an additive;
wherein the pH of the emulsion is greater than about 7; and
wherein the mean droplet size of the emulsion is less than about 200 nm.

2. The emulsion of claim 1 wherein the substituted phenylacetic acid ester compound is present at from about 0.25 wt% to about 25 wt%.

3. The emulsion of any one of claims 1 or 2 wherein the water-immiscible solvent is present at from about 0.1 wt% to about 50 wt%.

4. The emulsion of any one of claims 1 to 3 wherein the tonicity modifier is present at from about 0.1 wt% to about 2.5 wt%.

5. The emulsion of any one of claims 1 to 4 wherein the pH buffering agent is present at from about 0.01 wt% to about 10 wt%.

6. The emulsion of any one of claims 1 to 5 wherein the pH buffering agent is sodium phosphate, sodium citrate, sodium bicarbonate, L-histidine, or TRIS.

7. The emulsion of any one of claims 1 to 6 wherein the stabilizer is present at from about 0.001 wt% to about 5 wt%.

8. The emulsion of any one of claims 1 to 7 comprising:
from about 1 wt% to about 10 wt% [3-ethoxy-4-[(*N,N*-diethylcarbamido)methoxy]phenyl]acetic acid *n*-propyl ester;
from about 5 wt% to about 15 wt% medium chain trigylceride;
from about 0.01 wt% to about 5 wt% soybean-derived lecithin;
from about 0.01 wt% to about 3 wt% glycerol; and
from about 0.001 wt% to about 1 wt% oleic acid.

9. The emulsion of any one of claims 1 to 8 comprising:
from about 3 wt% to about 9 wt% [3-ethoxy-4-[(*N,N*-diethylcarbamido)methoxy]phenyl]acetic acid *n*-propyl ester;
from about 6 wt% to about 12 wt% medium chain trigylceride;
from about 0.3 wt% to about 3 wt% soybean-derived lecithin;
from about 0.1 wt% to about 1 wt% L-histidine;
from about 0.001 wt% to about 0.1 wt% disodium edetate; and
from about 1 wt% to about 2.5 wt% glycerol.

10. The emulsion of any one of claims 1 to 9 further comprising a sedative hypnotic agent, an analgesic, or a paralytic agent.

11. The emulsion of claim 10 wherein the analgesic is an opioid.

12. An emulsion as claimed in any one of claims 1 to 11 for the treatment of inducing or maintaining anesthesia or sedation in a mammal.

## Patentansprüche

1. Injizierbare Emulsion, umfassend:
eine substituierte Phenylessigsäureesterverbindung, bei der es sich um [3-Ethoxy-4-[(*N,N-*diethylcarbamido)methoxy]phenyl]essigsäure-*n*-propyl-ester handelt;
ein Triglycerid mittlerer Kettenlänge in einer Menge von 5 Gew.-% bis 15 Gew.-%;
einen Emulgator, bei dem es sich um Sojabohnenlecithin handelt;
eine die Tonizität modifizierende Substanz und Wasser;
und gegebenenfalls weiterhin umfassend einen oder mehrere der Folgenden, ausgewählt aus einem den pH-Wert puffernden Mittel, einem Stabilisator und einem Additiv;
wobei der pH-Wert der Emulsion größer als etwa 7 ist und
wobei die mittlere Tröpfchengröße der Emulsion unter etwa 200 nm liegt.

2. Emulsion nach Anspruch 1, wobei die substituierte Phenylessigsäureesterverbindung in einer Menge von etwa 0,25 Gew.-% bis etwa 25 Gew.-% vorliegt.

3. Emulsion nach Anspruch 1 oder 2, wobei das nicht mit Wasser mischbare Lösungsmittel in einer Menge von etwa 0,1 Gew.-% bis etwa 50 Gew.-% vorliegt.

4. Emulsion nach einem der Ansprüche 1 bis 3, wobei das die Tonizität modifizierende Mittel in einer Menge von etwa 0,1 Gew.-% bis etwa 2,5 Gew.-% vorliegt.

5. Emulsion nach einem der Ansprüche 1 bis 4, wobei das den pH-Wert puffernde Mittel in einer Menge von etwa 0,01 Gew.-% bis etwa 10 Gew.-% vorliegt.

6. Emulsion nach einem der Ansprüche 1 bis 5, wobei es sich bei dem pH-Wert puffernden Mittel um Natriumphosphat, Natriumzitrat, Natriumhydrogencarbonat, L-Histidin oder TRIS handelt.

7. Emulsion nach einem der Ansprüche 1 bis 6, wobei der Stabilisator in einer Menge von etwa 0,001 Gew.-% bis etwa 5 Gew.-% vorliegt.

8. Emulsion nach einem der Ansprüche 1 bi 7, umfassend:
etwa 1 Gew.-% bis etwa 10 Gew.-% [3-Ethoxy-4-[(*N,N-*diethylcarbamido)methoxy]phenyl]essigsäure-*n*-propyl-ester;
etwa 5 Gew.-% bis etwa 15 Gew.-% eines Triglycerids mittlerer Kettenlänge;
etwa 0,01 Gew.-% bis etwa 5 Gew.-% Sojabohnenlecithin;
etwa 0,01 Gew.-% bis etwa 3 Gew.-% Glycerin und
etwa 0,001 Gew.-% bis etwa 1 Gew.-% Ölsäure.

9. Emulsion nach einem der Ansprüche 1 bis 8, umfassend:
etwa 3 Gew.-% bis etwa 9 Gew.-% [3-Ethoxy-4-[(*N,N-*diethylcarbamido)methoxy]phenyl]essigsäure-*n*-propyl-ester;
etwa 6 Gew.-% bis etwa 12 Gew.-% eines Triglycerids mittlerer Kettenlänge;
etwa 0,3 Gew.-% bis etwa 3 Gew.-% Sojabohnenlecithin;
etwa 0,1 Gew.-% bis etwa 1 Gew.-% L-Histidin;
etwa 0,001 Gew.-% bis etwa 0,1 Gew.-% Dinatrium-edetat und
etwa 1 Gew.-% bis etwa 2,5 Gew.-% Glycerin.

10. Emulsion nach einem der Ansprüche 1 bis 9, weiterhin umfassend ein sedatives Hypnotikum, ein Analgetikum oder ein Paralytikum.

11. Emulsion nach Anspruch 10, wobei es sich bei dem Analgetikum um ein Opioid handelt.

12. Emulsion nach einem der Ansprüche 1 bis 11 zur anästhesieinduzierenden oder -aufrechterhaltenden Behandlung oder Sedation eines Säugetiers.

## Revendications

1. Émulsion pour injection comprenant :
un ester d'acide phénylacétique substitué qui est le [3-éthoxy-4-[(*N,N*-diéthylcarbamido)-méthoxy]phényl]acétate de *n*-propyle ;
un triglycéride à chaîne moyenne présent à une teneur comprise entre 5 % et 15 % en masse ;
un émulsifiant qui est une lécithine dérivée du soja ;
un modificateur de tonicité ; et
de l'eau ;
et éventuellement comprenant en outre un ou plusieurs des agents choisis dans le groupe suivant : agent tamponnant le pH, stabilisant et adjuvant ;
le pH de l'émulsion étant supérieur à environ 7 ; et
la taille moyenne des gouttelettes de l'émulsion étant inférieure à environ 200 nm.

2. Émulsion conforme à la revendication 1, où l'ester d'acide phénylacétique substitué est présent à une teneur comprise entre environ 0,25 % en masse et environ 25 % en masse.

3. Émulsion conforme à l'une quelconque des revendications 1 ou 2, où le solvant immiscible à l'eau est présent à une teneur comprise entre environ 0,1 % en masse et environ 50 % en masse.

4. Émulsion conforme à l'une quelconque des revendications 1 à 3, où le modificateur de tonicité est présent à une teneur comprise entre environ 0,1 % en masse et environ 2,5 % en masse.

5. Émulsion conforme à l'une quelconque des revendications 1 à 4, où l'agent tamponnant le pH est présent à une teneur comprise entre environ 0,01 % en masse et environ 10 % en masse.

6. Émulsion conforme à l'une quelconque des revendications 1 à 5, où l'agent tamponnant le pH est le phosphate de sodium, le citrate de sodium, le bicarbonate de sodium, la L-histidine ou le TRIS.

7. Émulsion conforme à l'une quelconque des revendications 1 à 6, où le stabilisant est présent à une teneur comprise entre environ 0,001 % en masse et environ 5 % en masse.

8. Émulsion conforme à l'une quelconque des revendications 1 à 7, comprenant :
entre environ 1 % en masse et environ 10 % en masse de [3-éthoxy-4-[(*N,N-*diéthylcarbamido)méthoxy]phényl]acétate de *n*-propyle ;
entre environ 5 % en masse et environ 15 % en masse de triglycéride à chaîne moyenne ;
entre environ 0,01 % en masse et environ 5 % en masse de lécithine dérivée de soja ;
entre environ 0,01 % en masse et environ 3 % en masse de glycérol ; et
entre environ 0,001 % en masse et environ 1 % en masse d'acide oléique.

9. Émulsion conforme à l'une quelconque des revendications 1 à 8, comprenant :
entre environ 3 % en masse et environ 9 % en masse de [3-éthoxy-4-[(*N,N-*diéthylcarbamido)méthoxy]phényl]acétate de *n*-propyle ;
entre environ 6 % en masse et environ 12 % en masse de triglycéride à chaîne moyenne ;
entre environ 0,3 % en masse et environ 3 % en masse de lécithine dérivée de soja ;
entre environ 0,1 % en masse et environ 1 % en masse de L-histidine ;
entre environ 0,001 % en masse et environ 0,1 % d'édétate de disodium ; et
entre environ 1 % en masse et environ 2,5 % en masse de glycérol.

10. Émulsion conforme à l'une quelconque des revendications 1 à 9, comprenant en outre un agent hypnotique sédatif, un analgésique ou un agent paralytique.

11. Émulsion conforme à la revendication 10, où l'analgésique est un opioïde.

12. Émulsion conforme à l'une quelconque des revendications 1 à 11 destinée au traitement consistant en l'induction ou en la conservation de l'anesthésie ou de la sédation chez un mammifère.
